(19)

Europäisches
Patentamt
European
Patent Office
Office européen
des brevets

(11) **EP 1 281 067 B1**

(12) **EUROPEAN PATENT SPECIFICATION**

(45) Date of publication and mention
of the grant of the patent:
**17.01.2007 Bulletin 2007/03**

(51) Int Cl.:
***G01N 25/48*** (2006.01)

(21) Application number: **01933482.0**

(22) Date of filing: **08.05.2001**

(86) International application number:
**PCT/BE2001/000080**

(87) International publication number:
**WO 2001/085978 (15.11.2001 Gazette 2001/46)**

(54) **METHOD FOR HIGH-THROUGHPUT LEAD PROFILING**

VERFAHREN ZUR HOCHDURCHSATZPROFILIERUNG NEUER WERKSTOFFE

PROCEDE ET TECHNIQUE DE PROFILAGE HAUT DEBIT D'UNE TETE DE SERIE

(84) Designated Contracting States:
**AT BE CH CY DE DK ES FI FR GB GR IE IT LI LU
MC NL PT SE TR**

(30) Priority: **08.05.2000 US 202475 P**

(43) Date of publication of application:
**05.02.2003 Bulletin 2003/06**

(73) Proprietor: **Vivactis NV
3001 Leuven (BE)**

(72) Inventors:
• **VERWAERDE, Philippe
F-59960 Neuville en Ferrain (FR)**
• **VERHAEGEN, Katarina
B-3000 Leuven (BE)**

(74) Representative: **Bird, Ariane et al
Bird Goen & Co c.v.
Klein Dalenstraat 42A
3020 Winksele (BE)**

(56) References cited:
**EP-A- 0 921 391          WO-A-00/26651
WO-A-99/49973          WO-A-99/53094
US-A- 5 255 976          US-A- 5 654 150
US-A- 5 842 788**

• **MAYER G ET AL: "NANOTITERPLATES FOR
SCREENING AND SYNTHESIS" BIOMETHODS,
BIRKHAEUSER, BASEL, CH, 1999, pages 75-128,
XP000911754 ISSN: 1018-6255**
• **HAROLD J ET AL: "MEMS TECHNOLOGY IS
POISED FOR WIDE-SCALE
COMMERCIALIZATION" ELECTRONIC DESIGN,
PENTON PUBLISHING, CLEVELAND, OH, US, vol.
45, no. 11, 27 May 1997 (1997-05-27), pages
121-122,124,126,128,130,132, XP000724051
ISSN: 0013-4872**

EP 1 281 067 B1

## Description

### Field of the invention

The present invention is related to a method for high-throughput lead profiling and the use of microcalorimetric devices therein.

### State of the art

In response to increased economic pressures, research-based pharmaceutical companies need to accelerate their drug discovery and development processes. The most critical factor in the development of new significant NCEs is the preclinical testing, in which an average of sixty percent of NCEs produced by drug discovery fail. Of the forty percent that eventually undergo human clinical testing, less than one tenth ultimately come through clinical trials and become marketed products. Thus, the amount of lead compounds that are optimized to development candidates and that eventually reach early clinical trials is still alarmingly high.

Most of the failures are due to undesired or unpredicted interactions with non-specific targets. This can result in bad or no transport across membranes, rejection of drugs by Multiple Drug Resistance Proteins or P-glycoproteins, transformation or destruction of the drug by metabolic enzymes, toxicity as drugs affect essential targets for cell survival, side effects of the drugs caused by interaction with unpredicted receptors or enzymes.

The high level of attrition in compounds developed as leads has prompted many companies into applying technologies such as high throughput target profiling in early drug discovery, integrated in a strategy to assess the "drug-like characteristics" of chemical leads, with the object of predicting and avoiding those failures that occur in clinical trials. At the same time these strategies and technologies can also be used in the optimization of chemical leads into NCEs.

This pharmaceutical profiling, integrating ADME and pharmacokinetic information into early and mid-discovery, optimally includes analysis of drug solubility and permeability (commensurate with route(s) of drug administration.), stability, metabolism liabilities, pharmacokinetic parameters including blood concentrations, distribution and half-life, appropriate formulation excipients and toxicity and undesirable side effects.

Toxicity studies are performed mainly by assaying the chemical entity (hits, leads or drugs) on a panel of different receptors and enzymes, optionally also on whole-cell systems. A limited number of companies offer lead profiling, some for up to 300 targets. However, the maintenance and updating of hundreds of different assays on many targets is very costly. At the same time the generation of a representative number of targets that can be screened is a problem. The ability to do profiling of new chemical entities against all (or at least a large number of) potential targets however, would increase the chance of identifying unpredicted interactions. Additionally, the availability of a screening method which allows the (rapid) identification of an interaction between a chemical entity and large number of targets could dramatically reduce drug development failures by enabling pharmaceutical lead profiling at an early stage.

The most sensitive assays for determining interaction (i.e. binding and/or activity) of a chemical with a target rely on radioactivity- or fluorescence-based detection methods. These methods have obvious disadvantages when working with small molecules and/or living cells. Alternatively, most physiometers do not allow sensitive testing on a high-throughput scale.

The present invention discloses a method to perform high throughput screening of chemical entities for their activity on multiple targets, based on micro-calorimetry.

Calorimetry in general is a measurement principle that detects all processes that occur in a reaction vessel. Calorimetry has several advantages: calorimetry is the most general detection principal, since most processes, physical, chemical or biological are accompanied by changes in heat content. This can be useful in the analysis of very complex processes, because it is more likely that unknown phenomena will be discovered. Moreover, the change of temperature of a reaction volume is dependent upon the concentration of the reagents, not upon the absolute quantity, offering the possibility of miniaturisation. Additionally, calorimetric methods are not dependent upon the sample form (the sample can be solid, liquid, gaseous, or any combination thereof, and neither colour, optical transparency, nor absence of suspended matter are requirements). Calorimetric measurements are non-invasive (it is not necessary to disturb the biological system, for example, by radiation). It even allows on-line monitoring of living cells over a longer period of time to obtain kinetic data. It is non-destructive towards the sample (no need for fluorescent markers or fixation procedures).

The state of the art are devices capable of measuring small temperature differences between relatively large reaction volumes (typically 0.12 ml to 100 ml). SETARAM(Caluire, France) is a provider of calorimetric devices. These products are relatively large batch systems, mainly intended for applications in the biochemical field. The ITC® (Isothermal Titration Calorimeter, MicroCal, LLC, Northampton, USA) has a cell volume of 1.3 ml (and a detection limit of 400 nW). TheTAMED (Thermal Activity Monitor, Thermometric, Jarfalla, Sweden) has a cell volume of 4 ml (and a detection limit of approximately 100 nW). These micro-calorimeter batch systems are non-compatible with high-throughput requirements due to the relatively large volumes, the apparatus closed structure and long cycle times (typical cycle times are 2 to 3 hours).

other devices known in the art are capable of detecting small absolute temperature changes in extremely small reaction volumes. Commercially available microcalorimetric sensors have also been described, using thermopiles and thermistors (Xensor Integration, Delft, The Netherlands). A main disadvantage of these devices is that they are configured in such a way that the reference temperature is the temperature of the silicon border. This leads to problems concerning baseline stability and common rejection mode since no differential measurements are used. Additionally, these devices are not compatible with standard robotics used in high throughput screening since no recipients are integrated. US-5255976 discloses a temperature gradient apparatus with two-dimensional data collection capability for monitoring both intermolecular and intramolecular binding reactions.

The apparatus disclosed consists of an array of wells in a substrate, which is a plate formed from a block of thermally conductive material into which wells are formed.

A temperature gradient is produced across the substrate and the array. The extent of reaction taking place in a given well is dependent on temperature and concentration of an added agent. Binding of a reactant agent to a protein in a sample of reactant solution according to this document can be detected by sending the corresponding change in fluorescence intensity, by measuring radioactively labelled materials, by measuring luminescence, by measuring the absorption of ultraviolet radiation or by measuring the absorption of visible radiation. From these measurements heats of reactions can be derived, Therefore, this document does not disclose a true calorimetric device as is meant in the present invention, i.e. where a direct heat measurement is performed.

US-5654150 discloses the generation of cDNA expression libraries and the subsequent assaying of these expression libraries by various assaying methods, including the use of the binding characteristics of proteins.

## Aims of the invention

The present invention aims to provide a new method for high-throughput screening of chemical entities on a large number of targets.

## Summary of the invention

The present invention comprises a method for high-throughput screening of chemical entities on a large number of targets, as defined in claim 1.

According to a specific embodiment of the present invention the large number of targets encompasses a collection of cells or cellular proteins.

According to one aspect of the invention said method comprises generating a cDNA expression library of an organism in a host and testing the chemical entity against individual clones of this expression library. According to a preferred embodiment of the invention the cDNA expression library is a human cDNA expression library.

According to the invention the method comprises screening for the interaction of the chemical entity and the target by way of calorimetry. In a preferred embodiment said calorimetric measurement is performed using a microcalorimetric device comprising a differential heat detection means. Most preferably this differential heat detection means is a thermopile.

An aspect of the present invention is a calorimetric measuring method for measuring the interaction of a chemical entity with multiple targets for use in drug-profiling, said method comprising the steps of:

- Providing different targets in the reaction vessels of a microcalorimetric device,
- Adding the chemical entity to the targets in the test reaction vessels, and,
- Measuring the heat released by the interaction between the chemical entity and the targets using a microcalorimetric device. Said microcalorimetric device is an array device comprising a substrate and at least two array elements that are separated from each other by a first thermal isolation zone, each element comprising a first and a second receiving zone, both arranged to provide a contact between the chemical entity and one of the targets, there being a second thermal isolation zone between the first and second receiving zone, wherein said first and second thermal isolation zones are formed by at least part of said substrate.

Preferably the calorimetric device is a device capable of measuring very small temperature increases in multiple reaction vessels simultaneously. Most preferably, the calorimetric device is an array device as described herein.

It will be understood that particular embodiments of the invention are described by the dependent claims cited herein.

## Short description of the drawings

Figure 1 represents how acDNA Library can be obtained.

Figure 2 represents a microcalorimetric device according to the present invention.

Figure 3 represents a microcalorimetric device wherein the membrane is part of the substrate.

Figure 4 represents an illustration of a screening operation using acDNA library.

## Detailed description of the invention

The present invention discloses a method for screening the interaction of a chemical entity on a large number of targets.

A chemical entity as used herein relates to a chemical molecule or compound (hits, leads or drugs) obtained in drug development. Preferably the chemical entity corresponds to a hit or lead which has not yet been tested in pre-clinical or clinical trials.

A target as used herein relates to a DNA, protein, multi-protein complex, cellular structure, cell organelle, cell or tissue or culture thereof with which a drug can have a direct interaction. Thus the term "target" as used herein in fact refers to "non-specific" or non-intended targets, i.e. other molecules than the selected target the interaction with which was used as a criteria for selection. Alternatively, such targets will also be referred to as "samples". Preferably, according to the present invention, the interaction of a chemical entity is screened on a maximal number of samples, corresponding to most or all possible non-specific targets with which the chemical entity, when administered to a human or animal as a drug can interact. Thus, the set of samples can comprise a variety of cells (i.e. in culture) or cellular structures from different organs. Such cells include cancer cells or cells that are genetically modified. Alternatively, a collection of proteins (such as, i.e., blood proteins) or receptors. According to a preferred embodiment of the present invention, a maximal amount of possible samples is screened by using a cDNA expression library to generate a collection of samples.

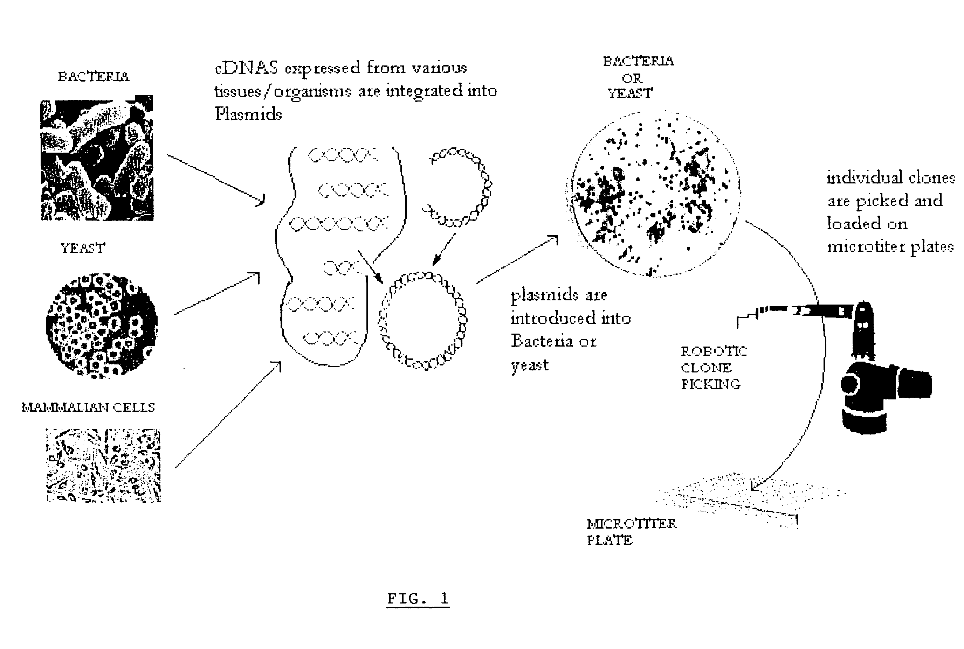

cDNA Libraries can be obtained according to standard recombinant DNA technology and essentially comprises the following steps: mRNA is prepared (characterized by a long poly-A tail at the 3' end, so it can be extracted using an oligo-dT column) after which oligo-dT primers are then annealed to the poly-A tail, which facilitates synthesis of a DNA copy through the use of reverse transcriptase. The original RNA strand is then removed by alkaline hydrolysis. DNA polymerase is used to synthesize the second DNA strand and this is initiated at various sites through the use of random hexamer nucleotide primers. Use of a DNA ligase covalently seals the remaining breaks. The cDNA may be inserted into a phage vector, such as lambdagt10, in order to create a library. These phage particles can be propagated through plaque formation on a bacterial lawn, alternatively cDNAs can be inserted in plasmids which are then introduced into bacteria or yeast by transformation. Genomes from various organisms from bacteria to man, including yeast and protozoa can be obtained from small isolates and subsequently expressed for screening. Genomes or cDNA libraries can be purchased separately from public banks (ATCC, etc...) or commercial suppliers. Each clone can be isolated and grown in a microplate format. Such a library can contain from thousands to hundreds of thousand isolates. Mother plates are replicated with a 96 or 384 pin tools. The replicated isolates can then be grown. The cells or bacteria can be disrupted with a specific lysis buffer or by ultrasound. Such protein lysates are then ready to be screened (Figure 1).

The present invention discloses a method for screening the interaction between a chemical entity and a large number of samples. For the purpose of this invention, the interaction between the chemical entity and the sample is referred to as an "event". Such an event can induce different signals such as change of heat or enthalpy, change of ionic concentrations of different ions, etc.... Thus, the occurrence of an event can be measured by pH-metry or calorimetric methods measuring heat and/or irradiation.

According to a preferred embodiment of the invention, the interaction is measured by calorimetry, more specifically by an apparatus capable of measuring small temperature changes generated in multiple reaction vessels, i.e. wherein multiple heat detection means are set up in an array. According to a preferred embodiment of the invention, the device for use in screening of the interaction between a chemical entity and multiple samples, is a device as described hereafter.

The apparatus comprises an array device, said array device comprising on a supporting substrate at least two array elements that are separated from each other by an isolation zone, said array elements comprising:

- A receiving zone arranged to provide a contact between a sample and a chemical entity,
- A heat detection means arranged to perform a measurement of heat between said receiving zone and a reference, and
- Said isolation zone being formed by at least part of said supporting substrate,
  Characterised in that said supporting substrate has sufficient strength to support said array device and said isolation zone is arranged to thermally isolate said array elements

The array device comprises a substrate with at least two substantially identical reaction vessels or receiving zones which preferably form a part of the substrate. The reaction vessel is used for retaining the chemical entity and for providing contact between the chemical entity and the protein or cell sample.

The device further comprises at least one heat detection means. Said heat detection means can be a heat detection means selected from the group consisting of a thermistor, diode, IR detection means, CCD camera, a thermopile. Preferably said heat detection means is a differential heat detection means integrated in the substrate for monitoring changes in heat content or enthalpy generated by the chemical entity upon being contacted with the sample. The differential heat detection means is operatively associated with the first and the second reaction vessel (or receiving zones) so that a differential measurement between the first reaction vessel (the "reference" reaction vessel) and the second reaction vessel (the "measurement" reaction vessel) can be performed. The first and the second reaction vessel are preferably neighbouring reaction vessels.

The differential heat detection means (DHDM) of the device is arranged to perform a differential measurement of heat between two reaction vessels (or receiving zones). The first receiving zone is used as a reference while in the other an event is generated. When a change of heat or enthalpy is generated, the thermal input signal is converted into a differential electrical output signal. Preferably, the reference sample is substantially equivalent to the test sample, so that heat capacities and surface relationships (which cause condensation or evaporation) do not influence the measurement.

The array device preferably has the format of a standard microtitre plate, such as, but not limited to a 96-well, 384-well, or 1536-well microtitre plate (see table 1). Alternatively, the design of the array can be customised for integration in any high-throughput screening system. This allows the use of the array in standard robotics used in drug screening. In a preferred embodiment of the invention, the array is a sensor-arrayed chip with the footprint of a standard 96-well titre plate, and thus compatible with pharmaceutical robotics for dispensing and titre plate handling. The distance between adjacent wells in this format is 9 mm. For formats derived from this reference, the inter-well distance is 9 mm divided by the miniaturisation factor. The miniaturisation factor is defined as

$$m = \sqrt{\frac{n\_wells}{96}}$$

with n_wells the number of wells.

The differential heat detection means in the device used according to a preferred embodiment of the invention is a thermopile, consisting of a set of 2 temperature sensitive means with a differential read out. Said thermopile has a hot and a cold junction, which are operatively associated with the first and the second reaction vessel respectively. The hot and cold junction are thermally isolated one from another.

A thermopile is made up of a number of thermocouples, electrically connected in series, thermally connected in parallel.

The main advantages of a thermopile are:

- The thermopile is a self-generating offset-less device, as the heat flowing through it supplies the power for the output signal. As a result there is no offset drift and no interference caused by power supplies.
- The sensitivity of the thermopile is hardly influenced by variations in the electrical parameters across the wafer or by the temperature.

The thermopile can be optimised in terms of dimension and number of thermo-electric strips.

The substrate of the device used according to a preferred embodiment of the invention can be, but is not limited to, one of the group consisting of silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber. When the substrate is a material compatible with semiconducting processing, the integration of sensors and fluidics on the same chip is facilitated. This opens the way to small sample volumes and inert reaction vessels, since no reaction with materials or adsorption of materials occurs. Said substrate should be made such that it has sufficient strength to support said array

The reaction vessel (or receiving zone) of the device used in the context of the present invention refers to a means or carrier of sample and/or chemical entity. More specifically, if the sample used is biological material, such as a cell culture, the reaction vessel will preferably be of a format capable of containing fluids. Preferably, the reaction vessel is capable of handling volumes in the range from 0 ml to 100 ml, more preferably from 0 ml to 7 ml, especially preferably from 0 ml to 5 ml, most preferably from 0.1 nl to 1 ml. Preferably, said reaction vessel has a maximum volume of 5 ml. Furthermore, the reaction vessel is operatively associated with the differential heat detection means. This means that there is a thermal coupling between the reaction vessel and the temperature sensitive part of the differential heat detection means.

In a preferred embodiment of the device used according to the present invention, the reaction vessel (or receiving zone) is formed by a recess in a substrate. Said reaction vessel can also be an area on the substrate e.g., the whole substrate or parts of the area of the substrate can be chemically or physically modified in such a way that they can selectively hold one or more of said sample, medium, and chemical stimulus.

Alternatively, the reaction vessel (or receiving zone) can be a microvessel made of a material, such as but not limited to, metal, steel, silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber, which is either placed on top of, or is hanging above the substrate. For instance, the reaction vessel can be formed by a needle form dispenser. Preferably, the distance between the substrate and the reaction vessel is not larger than the distance between two adjacent reaction vessels.

As described above, each reaction vessel (or receiving zone) is surrounded by an "isolation zone", which functions as a thermal isolation zone.

The membrane of the device used according to a preferred embodiment of the present invention is a part of the device which provides thermal isolation. The membrane can be part of the substrate or can be a second substrate.

Alternatively, when the reaction vessel is formed by complete etches through the substrate extending from the first surface to the second surface, a thin membrane can be formed for covering said recess at a first side or at the second side of the substrate.

The membrane can be made of the substrate material or of another material. The membrane can be formed, by methods such as, but not limited to, growing a membrane layer on the substrate or bonding a membrane.

The membrane can also be made of another material than the substrate material. For instance, a thin layer of the membrane material can be formed on the substrate material. The recess can be formed in the substrate by conventional micromachining techniques. The recess can extend from the first surface of the substrate until the membrane. When e.g. dry etching techniques are used, the membrane material and etch chemistry can be chosen such that a recess is etched in the substrate and that the etching process selectively stops on the membrane.

The thickness of the membrane can be between 1 $\mu$m and 1 cm, preferably between 1$\mu$m and 1 mm, most preferably between 10 $\mu$m and 0.1 mm. The thickness depends on the membrane material, and consequently on the thermal isolation of the membrane material. For example, the thickness of a silicon oxide membrane can be lower than 10 $\mu$m. For glass, the thickness can be between 1 and 5 mm.

Any of the above mentioned materials can be perforated and/or patterned material such that a perforated membrane or sieve is created. When a liquid is supplied to the reaction vessel, oxygen molecules can escape through these openings. Additionally or alternatively, the sieve serves to encapsulate biological material. Sieves are useful when specific elements, like cells or tissue, must be entrapped inside the physiometer, while maintaining (periodic) contact with a nutritive medium.

The size of the openings of the sieve is determined by the function of the sieve. For instance, when desiring cellular entrapment, the openings will preferably have a diameter of about 6 $\mu$m, while for tissue entrapment the openings will preferably have a diameter of about 20 $\mu$m. It is understood that the size of the openings can be optimised depending on the sample used.

The sieve is preferably made of low stress material (1.2 $\mu$m plasma oxide). In places where sensors are positioned, other structural layers are present.

When the reaction vessel is a recess in the substrate, the openings will extend from the bottom of the recess to the second surface of the substrate. When the reaction vessel is part of the first surface, the openings will extend from this first surface to the second surface. Said membrane can also be a suspended membrane.

The device or array of devices can further comprise a membrane which covers it. This membrane can also be called "lid". The lid can be made of at least one material such as, but not limited to, metal, steel, silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber.

The same sieve structure as described for the membrane can be found in the lid. The lid can cover the reaction vessel. In this way, cell or tissue can be entrapped.

The device used according to a preferred embodiment of the invention, can further comprise an extra detection means for screening the interaction between a chemical entity and the samples. This extra detection means can be a potentiometric sensor (such as, but not limited to, a Light Adressable Potentiometric sensor or "LAPS"), a FET device, a diode, interdigitated electrodes ("IDES"), a reference electrode, a working electrode and/or an impedance spectroscopic device. This extra detection means can be placed such that it is operatively associated with said reaction vessel. For example, said extra detection means can be integrated in the substrate. When the reaction vessel is a recess in the substrate, the extra detection means can be integrated in e.g., the side walls or the bottom wall of the reaction vessel. When the reaction vessel is an area on the substrate, the extra detection means is preferably integrated in the substrate. The number of extra detection means per reaction vessel is not limited.

Furthermore, in the context of the present invention it is envisaged that, in particular circumstances in determining the interaction between a chemical entity and a sample, it may be required to set the device at a given temperature. Therefore, the device used in the context of the present invention may further comprise a calibration means for thermosetting said device. The calibration means can be a tuneable electrical power generating means such as e.g. a resistor or a thermopile or a temperature sensitive means such as a resistor, diode, thermopile or a microprocessor that drives the power generator in such a way that a pre-determined temperature is obtained.

Additionally, the device used in the context of the invention can comprise a supply means to fill the reaction vessels in an active (e.g. pressure or suction force) or passive (e.g. capillary force) manner. The supply means can be fabricated in a material such as, but not limited to silicon, silicon oxide, silicon nitride, silicon oxynitride, polysilicon, porous silicon, plastic, polymer (also rubber, PVC, etc...), biodegradable polymer, glass, quartz, ceramics, aluminium oxide, agar, biological material, and rubber. In a preferred embodiment of the invention, the supply means has the same dimensions and layout as the device, except the heat detection means is omitted. The supply means can optionally have external or internal (e.g. made by micromachining) pumps and valves. Alternatively, said supply means can also be an industrial dispenser with or without external pumps and valves. The supply means can also be a custom-made dispenser with or without external or internal (e.g. made by micromachining) pumps and valves.

Optionally, the apparatus used in the context of the present invention comprising an array of devices makes use of capillary forces to supply the solutions to the reaction vessel(s).

The device of the present invention can further comprise read-out electronics such as (pre-) amplifying, multiplexing, filtering and/or buffering circuitry.

For calibrating said the calorimetric measurement, resistors, other than those used for thermosetting, are used as calibration means and are situated in the walls of the reaction vessels. The calibration resistor is designed to be able to produce dissipation heat in the same range as the produced biological power.

Techniques used to fabricate such an array device can be, but are not limited to, molding or micro-electronic processing. Micro-electronic processing offers specific advantages such as cost reduction if mass production is envisioned. Further, extreme miniaturisation is possible, the degree of which is limited by e.g. lithography. A high degree of parallelism is possible, making high-throughput feasible. Moreover, the detection limit of the system can be decreased considerably because fluidics are integrated on the same chip as the sensing element, reducing the length of the thermal path from reaction to sensing site. In addition, the detection limit of the system can be decreased even more if pre-amplifiers are integrated on the chip.

**Example: Identification of proteins interacting with a compound issued from a pharmacological screening**

Compound C is selected as a lead molecule, in a drug development proces involving the screening of a commercial compound library for specific interaction with a target, receptor R.

In order to obtain information on the possible interaction of this compound with non-intended targets, it is screened against proteins obtained from an animal or human expression library. Interaction between the compound and proteins is measured via microcalorimetry. Once positives have been identified, the corresponding cDNA is sequenced.

a) development of a sample library

A cDNA library is prepared from DNA isolated from human cells according to classical recombinant DNA technology and introduced in a phage vector in a bacterial host. Each clone is isolated and grown in a microplate format. Mother plates are replicated with 96- or 384- pin tools. The replicated isolates can then be grown. The bacteria are disrupted with a specific lysis buffer or by ultrasound. The protein lysates so obtained can be used for screening (Figure 1).

b) Screening

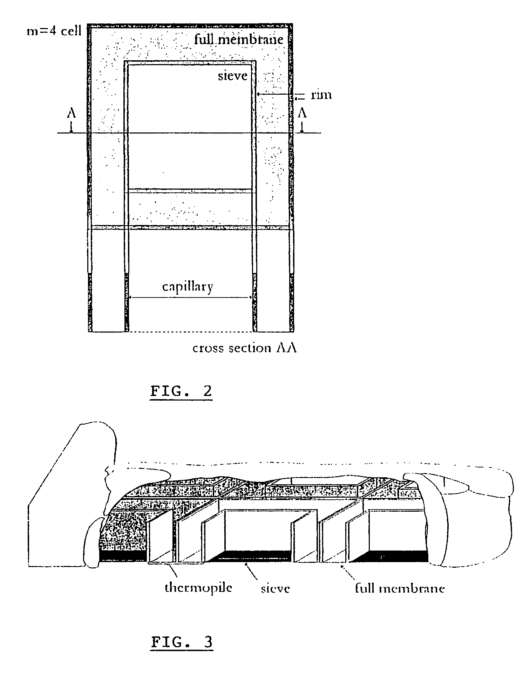

Screening is performed by way of a microcalorimeter based on a device which allows the detection of extremely small temperature differences between two reaction vessels, which are biocompatible and of which an array can be formatted which has the footprint of a microtitre plate (Figure 2).

The use of silicon as substrate material provides the possibility of integrating the sensors and fluidics on the same chip, so that sample volumes can be minimalized (minimal dead volume). Additionally, the reaction vessels which are recesses in the substrate are inert, as no reaction or sorption of materials occurs. As differential heat detection means a thermopile is integrated in the substrate. Additionally, 2 resistors are integrated in the walls of the reaction vessels as thermosetting means. The membrane is perforated to obtain a sieve with openings 6 $\mu$m wide, to enable cellular entrapment.

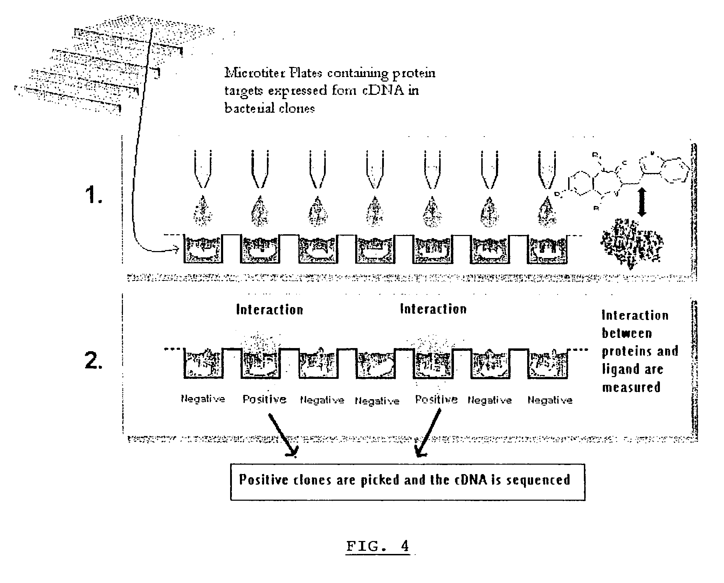

The measurement is performed essentially as follows (Figure 4):

- All reference reaction vessels (blanks) of the microtitre plate contain only screening buffer
- All measurement reaction vessels contain the compound C (prepared in a screening buffer)
- Temperature is monitored in real time.
- A number of measurement reaction vessels are each loaded with a sample of protein lysate obtained from a clone of the cDNA expression library, containing (as of yet) unidentified proteins. Control reaction vessels are loaded with the receptor R
- Temperature is monitored in real time.

Alternatively, the protein and reference samples are loaded in the reaction vessels first, and the chemical entity is added after a first real time temperature measurement.

Presence of a protein in sample of the protein lysate which interacts with compound C causes an interaction. The reaction enthalpy heats up the reaction vessel and this is detected by the DHDM. Additionaly, any accompanying change in proton concentration is detected by the pH detector. The coordinates of the well in which a thermal signal is detected allow the identification of the corresponding bacterial clone.

c) Hit processing

Positive reaction vessel coordinates are registered into a database; the corresponding cDNA is then automatically sequenced. The DNA sequenced is then analyzed using bioinformatics softwares to identify the corresponding amino acid sequence, which is finally compared to existing information on public databases. This information can be

- the identity of the protein
- the function of the protein
- the hypothetical function of the protein based on homology with a known amino acid sequence.

**Claims**

1. A method for high-throughput screening of chemical entities on a large number of samples, said method comprising the steps of:

a) providing different samples in measurement reaction vessels of a microcalorimetric device,
b) adding the chemical entity to the samples in reaction vessels, and
c) measuring the heat released by the interaction between the chemical entity and the samples using a microcalorimetric device, **characterized in that** said micro calorimetric device is an array device comprising a substrate and at least two array elements that are separated from each other by a first thermal isolation zone, each element comprising a first and a second receiving zone, both arranged to provide a contact between the chemical entity and one of the samples, there being a second thermal isolation zone between the first and second receiving zone, wherein said first and second thermal isolation zones are formed by at least part of said substrate.

2. The method according to claim 1, **characterised in that**

- the different samples comprise a cDNA expression library of an organism in a host, and
- the chemical entity is tested for the interaction with the protein expression products of individual clones of the expression library,

wherein said interaction between said chemical entity and said samples is measured by way of calorimetry.

3. The method of claim 2, **characterized in that** said cDNA expression library is a human cDNA expression library.

4. The method of claim 2, **characterized in that** said host is at least one bacterium.

5. The method of any of the previous claims, **characterised in that** each array element comprises a heat detection means.

6. The method of claim 5, **characterized in that** said array elements comprise:

- a reference reaction vessel and a measurement reaction vessel with a cross-section of less than 10 mm,
- a heat detection means arranged to perform a measurement of heat between said measurement reaction vessel and said reference reaction vessel.

7. The method of any of claims 5 or 6, **characterized in that** said heat detection means is a differential heat detection means.

8. The method of claim 7, **characterized in that** said differential heat detection means is a thermopile.

9. The method of claim 8, **characterized in that** said thermopile comprises a plurality of thermocouples which are electrically connected in series and thermally connected in parallel.

10. The method of any of claims 6 to 9, **characterized in that** said reaction vessels further comprise a resistor.

11. The method of claim 6, **characterized in that** said heat detection means comprises at least two temperature sensitive devices and has a differential read-out

12. The method according to any of the previous claims, **characterized in that** the method comprises determining the pharmacological profile of the chemical entity.

13. The method according to claim 12, **characterized in that** said chemical entity comprises at least one chemical molecule.

14. The method according to claim 13, **characterized in that** said chemical entity is a hit, lead or drug.

15. The method of any of the previous claims, **characterized in that** said substrate is configured to support said array device.

16. The method of any of the previous claims, **characterized in that** the microcalorimetric device is dimensioned as a well plate.

**Patentansprüche**

1. Verfahren zum Screening mit hohem Durchsatz von chemischen Entitäten für eine große Anzahl von Proben, wobei das Verfahren die folgenden Schritte umfasst:

a) Bereitstellen unterschiedlicher Proben in Mess-Reaktionsgefäßen einer mikrokalorimetrischen Vorrichtung,
b) Zugeben der chemischen Entität zu den Proben in den Reaktionsgefäßen, und
c) Messen der Wärme, die durch die Interaktion zwischen der chemischen Entität und den Proben freigesetzt wird, unter Verwendung einer mikrokalorimetrischen Vorrichtung, **dadurch**

gekennzeichnet, dass die mikrokalorimetrische Vorrichtung eine Arrayvorrichtung ist, die ein Substrat und mindestens zwei Arrayelemente umfasst, die voneinander durch eine erste thermische Isolierzone getrennt sind, wobei jedes Element eine erste und zweite Aufnahmezone umfasst, die beide so angeordnet sind, dass sie einen Kontakt zwischen der chemischen Entität und einer der Proben bereitstellen, wobei eine zweite thermische Isolierzone zwischen der ersten und zweiten Empfangszone vorliegt, wobei die ersten und zweiten thermischen Isolierzonen aus zumindest einem Teil des Substrats gebildet sind.

2. Verfahren gemäss Anspruch 1, **dadurch gekennzeichnet, dass**

   - die unterschiedlichen Proben eine cDNA-Expressionsbibliothek eines Organismus in einem Wirt umfassen, und
   - die chemische Entität auf die Interaktion mit den Proteinexpressionsprodukten einzelner Klone der Expressionsbibliothek untersucht werden,

   wobei die Interaktion zwischen der chemischen Entität und den Proben mittels Kalorimetrie gemessen wird.

3. Verfahren gemäss Anspruch 2, **dadurch gekennzeichnet, dass** die cDNA-Expressionsbibliothek eine menschliche cDNA-Expressionsbibliothek ist.

4. Verfahren gemäss Anspruch 2, **dadurch gekennzeichnet, dass** der Wirt mindestens ein Bakterium ist.

5. Verfahren gemäss einem der vorhergehenden Ansprüche, **dadurch gekennzeichnet, dass** jedes Arrayelement eine Wärmenachweiseinrichtung umfasst.

6. Verfahren gemäss Anspruch 5, **dadurch gekennzeichnet, dass** die Arrayelemente umfassen:

   - ein Referenz-Reaktionsgefäß und ein Mess-Reaktionsgefäß mit einem Querschnitt von weniger als 10 mm,
   - eine Wärmenachweiseinrichtung, die so angeordnet ist, um eine Messung der Wärme zwischen dem Mess-Reaktionsgefäß und dem Referenz-Reaktionsgefäß durchzuführen.

7. Verfahren gemäss einem der Ansprüche 5 oder 6, **dadurch gekennzeichnet, dass** die Wärmenachweiseinrichtung eine differentielle Wärmenachweiseinrichtung ist.

8. Verfahren gemäss Anspruch 7, **dadurch gekennzeichnet, dass** die differentielle Wärmenachweiseinrichtung eine Thermosäule ist.

9. Verfahren gemäss Anspruch 8, **dadurch gekennzeichnet, dass** die Thermosäule eine Vielzahl von Thermoelementen umfasst, die elektrisch seriell und thermisch parallel miteinander verbunden sind.

10. Verfahren gemäss einem der Ansprüche 6 bis 9, **dadurch gekennzeichnet, dass** die Reaktionsgefäße des Weiteren einen Widerstand umfassen.

11. Verfahren gemäss Anspruch 6, **dadurch gekennzeichnet, dass** die Wärmenachweiseinrichtung mindestens zwei temperaturempfindliche Vorrichtungen umfasst und einen differentiellen Auslesewert besitzt.

12. Verfahren gemäß einem der vorhergehenden Ansprüche, **dadurch gekennzeichnet, dass** das Verfahren das Bestimmen des pharmakologischen Profils der chemischen Entität umfasst.

13. Verfahren gemäss Anspruch 12, **dadurch gekennzeichnet, dass** die chemische Entität mindestens ein chemisches Molekül umfasst.

14. Verfahren gemäss Anspruch 13, **dadurch gekennzeichnet, dass** die chemische Entität ein Hit, ein Lead oder ein Arzneimittel ist.

15. Verfahren gemäss einem der vorhergehenden Ansprüche, **dadurch gekennzeichnet, dass** das Substrat so ausgestaltet ist, um die Arrayvorrichtung aufzunehmen.

16. Verfahren gemäss einem der vorhergehenden Ansprüche, **dadurch gekennzeichnet, dass** die mikrokalorimetrische Vorrichtung als Wellplatte dimensioniert ist.

## Revendications

1. Procédé de présélection à grande vitesse d'entités chimiques sur un grand nombre d'échantillons, ledit procédé comprenant les étapes de :

   a) approvisionnement de différents échantillons dans des récipients de réaction à mesurer, dans un dispositif de microcalorimétrie,
   b) ajout de l'entité chimique aux échantillons dans les récipients de réaction, et
   c) mesure de la chaleur dégagée par l'interaction entre l'entité chimique et les échantillons, au moyen d'un dispositif de microcalorimétrie, **caractérisé en ce que** ledit dispositif de microcalorimétrie est un dispositif formant groupement comprenant un substrat et au moins deux éléments de groupement, qui sont séparés l'un de l'autre par une première zone d'isolation thermique, chaque élément comprenant une première et une seconde zones réceptrices, toutes deux disposées de manière à assurer un contact entre l'entité chimique et l'un des échantillons, une seconde zone d'isolation thermique étant prévue entre les première et seconde zones réceptrices, dans lequel lesdites première et seconde zones d'isolation thermique sont constituées par au moins une partie dudit substrat.

2. Procédé selon la revendication 1, **caractérisé en ce que**

   - les différents échantillons comprennent une bibliothèque d'expressions d'ADN complémentaire d'un organisme dans un hôte, et
   - l'entité chimique est soumise à l'essai pour rechercher l'interaction avec les produits d'expressions de protéines de clones individuels de la bibliothèque d'expressions,

   dans lequel ladite interaction entre ladite entité chimique et lesdits échantillons est mesurée par calorimétrie.

3. Procédé selon la revendication 2, **caractérisé en ce que** ladite bibliothèque d'expressions d'ADN complémentaire est une bibliothèque d'expressions d'ADN complémentaire humain.

4. Procédé selon la revendication 2, **caractérisé en ce que** ledit hôte est au moins une bactérie.

5. Procédé selon l'une quelconque des revendications précédentes, **caractérisé en ce que** chaque élément du groupement comprend un moyen de détection de chaleur.

6. Procédé selon la revendication 5, **caractérisé en ce que** lesdits éléments du groupement comprennent :

   - un récipient de réaction de référence et un récipient de réaction mesurer, ayant une section transversale inférieure à 10 mm,
   - un moyen de détection de chaleur destiné à exécuter une mesure de la chaleur entre ledit récipient de réaction à mesurer et ledit récipient de réaction de référence.

7. Procédé selon la revendication 5 ou 6, **caractérisé en ce que** ledit moyen de détection de chaleur est un moyen de détection de chaleur différentielle.

8. Procédé selon la revendication 7, **caractérisé en ce que** ledit moyen de détection de chaleur différentielle est une thermopile.

9. Procédé selon la revendication 8, **caractérisé en ce que** ladite thermopile comprend une pluralité de thermocouples, qui sont électriquement montés en série et thermiquement montés en parallèle.

10. Procédé selon l'une quelconque des revendications 6 à 9, **caractérisé en ce que** lesdits récipients de réaction comprennent en outre une résistance.

11. Procédé selon la revendication 6, **caractérisé en ce que** ledit moyen de détection de chaleur comprend au moins deux dispositifs sensibles à la température et a une sortie différentielle de mesure.

12. Procédé selon l'une quelconque des revendications précédentes, **caractérisé en ce que** le procédé comprend la détermination du profil pharmacologique de l'entité chimique.

13. Procédé selon la revendication 12, **caractérisé en ce que** ladite entité chimique comprend au moins une molécule chimique.

14. Procédé selon la revendication 13, **caractérisé en ce que** ladite entité chimique est une piste, une tête de série ou un médicament.

15. Procédé selon l'une quelconque des revendications précédentes, **caractérisé en ce que** ledit substrat est conformé pour soutenir ledit dispositif formant groupement.

16. Procédé selon l'une quelconque des revendications précédentes, **caractérisé en ce que** le dispositif de microcalorimétrie est dimensionné comme une plaque à cavités.

FIG. 1

FIG. 2

FIG. 3

12

EP 1 281 067 B1

Microtiter Plates containing protein targets expressed fom cDNA in bacterial clones

**1.**

**2.**

Interaction     Interaction

Interaction between proteins and ligand are measured

Negative   Positive   Negative   Negative   Positive   Negative   Negative

Positive clones are picked and the cDNA is sequenced

FIG. 4